## Europäisches Patentamt
## European Patent Office
## Office européen des brevets

(19)

(11) Publication number: **0 114 759**
**A2**

(12) ## EUROPEAN PATENT APPLICATION

(21) Application number: **84300417.7**

(22) Date of filing: **24.01.84**

(51) Int. Cl.³: **C 07 C 103/52**
A 61 K 37/02, C 12 P 21/02
//A61K39/00, C12N15/00

(30) Priority: **24.01.83 GB 8301928**

(43) Date of publication of application:
**01.08.84 Bulletin 84/31**

(84) Designated Contracting States:
**BE CH DE FR GB IT LI LU NL SE**

(71) Applicant: **Nicholson, Bruce Heywood**
**Ashridgewood PLace Forest Road**
**Wokingham Berkshire, RG11 5RA(GB)**

(72) Inventor: **Nicholson, Bruce Heywood**
**Ashridgewood PLace Forest Road**
**Wokingham Berkshire, RG11 5RA(GB)**

(74) Representative: **Tubby, David George et al,**
**MARKS & CLERK 57-60 Lincoln's Inn Fields**
**London WC2A 3LS(GB)**

(54) **Amino acid sequences and polypeptides including these sequences and having the specificity of foot and mouth disease and other viral antigens.**

(57) An amino acid sequence or a synthetic polypeptide consisting of the same and preferably containing a helical structure is capable of being recognised by two or more antisera raised against different viral strains by virtue of the rotationally and translationally related different aspects presented by the helix to the antibodies. In particular, amino acid sequences of this invention may have rotationally related epitopic sites recognisable by different strains of foot and mouth disease virus.

EP 0 114 759 A2

0114759

M&C FOLIO: 47513 WANGDOC: 0072H

AMINO ACID SEQUENCES AND POLYPEPTIDES INCLUDING THESE
SEQUENCES AND HAVING THE SPECIFICITY OF FOOT AND MOUTH
DISEASE AND OTHER VIRAL ANTIGENS.

This invention relates to amino acid sequences, to polypeptides consisting of the same and to processes for producing these sequences and polypeptides, which have the specificity of two or more antigens of certain viruses and, in particular, of foot and mouth disease viral antigens.

The natural immune system is based upon the well known antigen-antibody reaction, in which an antigen (which is foreign to the host body - an example is a virus) stimulates the production by the host of antibodies specific to the antigen. These antigen-specific antibodies react with the antigen and, in a manner which is still being investigated, neutralize it. The antibodies so biosynthesized recognize specific "surface" features of the molecular structure of the antigen.

Antibodies to proteins and polypeptides can be conveniently classified into "conformational" and "sequential" types, according to whether they recognise the relatively static conformation of an antigenic determinant region, or an inherently more flexible sequence of amino acid residues. Most short, free polypeptides tend to lack any secondary structure sufficient to give a relatively static surface to form an antigenic determinant of the conformational type. However, in certain polypeptides where the amino acid residues have collectively sufficient helix-forming potential, the polypeptide can adopt a rigid helical structure in which the movement of the side chains of the amino acid residues is relatively spatially restricted. In this situation a specific antibody able to recognise the polypeptide would only be expected to do so when the helix was in the correct position with respect to the combining site of the antibody, that is, in a position such that those residues along the helix surface which collectively have the correct topology and charge distribution to form the antigenic determinant site are complementary to the combining site of the antibody. Expressed in geometrical terms, combination only occurs at the correct rotation about and translation along the helix axis with respect to the antibody.

Most viruses comprise a core of genetic material surrounded by a coat of protein or the like and it is this coat (or, more precisely, specific regions of the molecular structure of this coat) that antibodies recognize and neutralize. It is clear that an immune response to a given virus could be induced by injecting into the host a protein corresponding to the native coat protein or even a compound whose surface structure is or includes a section which is essentially the same as the antigenic determinant regions of that coat protein. Where an amino acid sequence forming part of the coat protein has adopted a helical configuration, some of the side chains on the amino acids making up that sequence will be directed into the interior of the protein as part of the hydrophobic core. It is only those side chains which are directed outwards and which are accessible to solvent that can be recognized by an antibody and are thus of importance when designing a synthetic compound to induce the same antigenic response.

Synthetic compounds of this type have the potential to be of considerable value, especially where disease control is currently by means of killed or attenuated virus vaccines, which can be potentially dangerous. However, most viruses exist as a number of strains and a vaccine effective against one strain may be ineffective or insufficiently effective against another. Accordingly, it would be necessary to manufacture separate compounds for each strain of the virus. EP 44710 discloses a possible solution to this problem by linking together in a single chain, which may be helical, two or more small peptide units, each unit containing a single antigenic determinant region.

Foot and mouth disease ("FMD"), also known as hoof and mouth disease, is a problem of major economic importance in many countries. Control of the disease is primarily with killed virus vaccines. A major problem with the production and application of the vaccine is the extreme variation found in the antigenic determinants on the FMD virus ("FMDV") particle responsible for eliciting an immune response.

Thus, FMDV exists as seven serotypes comprising more than 60 strains. Immunity to one serotype does not confer protection against the other six. Furthermore, control of the disease by vaccination is complicated by the extreme antigenic lability of the virus even within serotypes. The extent of this variation is such that vaccination will not necessarily give prolonged protection against other strains of the virus even within the same serotype. The antigenic variation between the serotypes is due to variation in the amino acid sequence of the VP1 capsid protein (which is the major coat protein of FMDV and which carries the major immunogenic portion of the coat), giving rise to structural variations in the sites (epitopes) which combine with antibodies. Similar problems exist with many other viruses, for example, the polio virus, or the viruses of influenza, hepatitis, viral hog cholera, rabies, smallpox, measles, rubella or mumps.

5          0114759

Two antigenic sites within VP1 have been
recognised. The first is around residues 138-154 and
the second is from 200 to the C terminal. The second is
probably a "sequential" type of determinant, but the
first is "conformational", and hence more suited for
secondary and tertiary structure analysis. In this
specification, as is conventional in the art, an amino
acid position in a given sequence is assigned a number
equating to the number of amino acid residues in the
sequence, counting from the N terminal to that
position. In considering synthetic sequences analogous
to a natural polypeptide or to part of a natural
polypeptide, it is generally convenient to base the
numbering system on the natural polypeptide and to use
that system for numbering the synthetic sequence, even
though the synthetic sequence may be different from the
natural sequence. Such differences may be of three
types:

(a) an amino acid at position "$\underline{x}$" of the natural
sequence may not be present in the synthetic
sequence - this is referred to as a "deletion at
position $\underline{x}$";

(b) an amino acid present in the synthetic sequence
may not have a counterpart in the natural sequence -
this is referred to as an "insertion between
positions $\underline{x}$ and $\underline{x + 1}$" of the natural sequence; and

(c) an amino acid at position $\underline{x}$ of the natural sequence may be replaced by a different amino acid - this is referred to as a "substitution at position x".

In the case of the VP1 protein of FMDV, the numbering is based upon a sequence identical to that of the VP1 protein of the $O_1K$ virus type, except that the $O_1K$ sequence has a deletion at position 199.

I have now discovered that it is possible to include 2 or more antigenic determinant regions on a single small polypeptide molecule, which may be helical, by arranging that the two or more regions are, in part or in whole, occupying the same parts of the molecule, e.g. the same turns of the helix. The amino acid sequence carrying those regions can be relatively small, e.g. from 7 to 30, more preferably from 10 to 20 and most preferably from 15 to 17, amino acid units long.

In the present invention, the sequence of amino acids which gives rise to one antigenic site is at least partially interpolated with other amino acids so that the conformation of the molecule provides at least two antigenic sites in which the amino acids of each site are conformationally contiguous, and said other amino acids forming at least part of one antigenic site.

Thus, the present invention consists in a molecule comprising an amino acid sequence having at least two antigenic regions, each said region being capable of being recognized by antibodies raised against a viral protein sequence, each said region being formed of a chain of amino acids of said sequence, the chains forming said regions having at least one amino acid (preferably more than one amino acid) of said sequence in common, so that the chains at least partly overlap and are not contiguous along the length of the molecule. The sequence is preferably such that it adopts a helical conformation, however, other conformations are possible, for example other sequences may form a structure similar to a ß-sheet.

The degree of overlap between the two (or more) antigenic regions and, hence, the chains forming them may vary considerably. Substantial overlap between the regions is clearly desirable, in order that the sequence may be as short as possible - thereby facilitating synthesis. For example, overlap such that at least 25% of the amino acids in one of said chains also form part of the other of said chains is preferred. An overlap of at least 50% is more preferred and a greater degree of overlap, e.g. at least 75%, may be aimed for, if possible. In some cases, it may be possible to achieve or, at least, approach 100% overlap.

The molecule of the invention is preferably a polypeptide.

The sequence of the invention is preferably coupled to a larger compound to form a single molecule, in order to facilitate antibody response, as is well known. Examples of such larger compounds include keyhole limpet haemocyamin, bovine serum albumin, btg, ß-galactosidase or penicillinase. The sequence of the invention may be covalently attached to the molecule of the larger compound or, especially where it and the larger compound are constructed together by recombinant DNA technology, the sequence of the invention may be linked in some other manner to the larger compound, for example, where the larger compound is a polypeptide such as ß-galactosidase or penicillinase, it may be interposed in the amino acid chain of that compound.

The molecules disclosed herein are characterised by having the specificity of two or more viral antigens, especially foot and mouth disease viral ("FMDV") antigens. As will be apparent from the disclosure to follow, the polypeptides, recombinant DNA molecules, and processes of this invention may be used in the production of antigenic polypeptides useful in diagnostic methods and in compositions for rendering animals resistant to FMDV for some period of time.

Secondary structure analysis of the amino acid sequence of VP1 protein of FMDV type $O_1K$ by the method of, for example, Chou and Fasman [Adv. Enzym. <u>47</u>, 45-148 (1978)] shows that a large part (144-160) of this sequence is helical, and furthermore that the corresponding sequence in the VP1 protein of FMDV type C3, which contains several amino acid substitutions compared to $O_1K$, is also helical. These sequences can also be analysed to see which groups are contiguous with the hydrophobic core of the VP1 protein. The basis of this method and the rules for one amino acid have been published [Nicholson, Biochem. Soc. Trans., <u>10</u>, 387 (1982)]. The method is based on the detailed analysis of the side-chain contacts of 24 proteins whose tertiary structure has been determined by X-ray crystallography, and the position of a particular residue with respect to the hydrophobic core of the protein correlated with the sequence of residues adjacent to it in the primary sequence, it being known that in nature the primary sequence determines the secondary and tertiary structure.

In FMDV type $O_1K$, all the residues in the core are on one side of the helix, except for Val 150, which would require a proximal side chain from elsewhere in the molecule to satisfy the core definition. A noticeable feature of the FMDV type $O_1K$ helix is the series of four polar side chains forming a "band" down one side which must be just above the surface of the contiguous protein, since the helix residues adjacent to the polar band residues are in the interior core of the rest of the protein.

The amino acid substitutions in the corresponding sequences of the different strains are shown in Table 1:

### TABLE 1

Epicylin

| | $P/O_1K/C3$ | $O_1K$ | A10 | A24 | C3 |
|---|---|---|---|---|---|
| 144 | Arg | LEU | Arg | Arg | Arg |
| 5 | Arg | Arg | Ser | Arg | Arg |
| 6 | Gly | Gly | Gly | Gly | Gly |
| 7 | Asp | Asp | Asp | Asp | Asp |
| 8 | Leu | Leu | Leu | MET | Leu |
| 9 | Gln | Gln | Gly | Gly | ALA |
| 150 | Thr | VAL | Ser | Thr | His |
| 1 | Leu | Leu | Leu | Leu | Leu |
| 2 | Ala | Ala | Ala | Ala | Ala |
| 3 | Gln | Gln | ALA | ALA | ALA |
| 4 | Lys | Lys | Arg | Arg | Ala |
| 5 | His | VAL | VAL | VAL | His |
| 6 | Ala | Ala | Ala | VAL | Ala |
| 7 | Arg | Arg | Thr | Lys | Arg |
| 8 | His | Thr | Gln | Gln | His |
| 9 | Leu | Leu | Leu | Leu | Leu |

In the above table and throughout this specification, the amino acids are identified by the internationally recognized standard three-letter amino acid code. The amino acids are underlined where they differ from those of Epicylin $P/O_1K/C3$ (which is a sequence in accordance with the present invention) and are in capitals where they are predicted to be in the protein core.

Their effect is as follows. In FMDV type C3, the Gln at 149 and 153 of $O_1K$ are replaced by Ala, both of which are now predicted to be in the core; Lys 154 is replaced by Ala (non-core); and some basicity is regenerated by the appearance of three His for two Val (150, 155) and Thr 158. Thus in FMDV type C3, and to some extent in types A10 and A24, the bulky polar side chains of the left hand side have been replaced by small hydrophobic side chains which are in the core and at the same time the right hand side has the two core Val replaced by non-core His. Effectively therefore, in the intact protein VP1 the helix viewed from the N-terminal has rotated anticlockwise with respect to the core, presenting a different topology for the constant features relative to the antibody binding sites, in addition to the substitutions themselves. The rotation may well be assisted by the deletion in the 140-145 range, which might explain why these residues appear to be involved in the antigenic response. Thus the antibody combining sites of the two epitopes are related by rotation about the helix axis.

The present invention utilises this discovery in developing synthetic polypeptides of appropriate amino acid sequence such that they form rotationally related epitopes which may interact with antibodies raised against different strains of virus and on coupling to suitable carrier proteins of natural or synthetic protein conventionally used for preparing antigens e.g. bovine serum albumin, induce antibody formation.

Also in Table 1, under the heading "Epicylin P/$O_1$K/C3", is shown an amino acid sequence in accordance with this invention and this has been found to elicit antibodies which neutralize virus type C3, as well as virus type $O_1$K, from which its structure is derived.  The amino acid sequence produced comprised the amino acid residues labelled 144-159.  It was synthesized by recombinant DNA technology by inserting a genetic sequence corresponding to the desired amino acid sequence between the EcoRl and BamHl sites of a ß-galactosidase gene in a modified plasmid vector (derived from pBR322) according to conventional methodology, and as illustrated subsequently in the Example.

Another amino acid sequence, Epicylin AP/A10/A12, was produced by chemical synthesis by inserting arginine between positions 145 and 146 of a sequence corresponding to that of the VP1 protein of virus type A10. The method employed was that of Bittle et al. [Nature 298, p 30-33 (1982)]. The structure of this Epicylin AP/A10/A12 is shown in Table 2, together, for comparison, with the corresponding sequences of VP1 protein of FMDV types A10, A12A and A12B.

TABLE 2

Epicylin

| | AP/A10/A12 | A10 | A12A | A12B |
|---|---|---|---|---|
| 144 | Arg | Arg | Val | Val |
| 145 | Ser | Ser | Arg | Arg |
| insert | Arg | -- | -- | -- |
| 146 | Gly | Gly | Gly | Gly |
| 147 | Asp | Asp | Asp | Arg |
| 148 | Leu | Leu | Ser | Leu |
| 149 | Gly | Gly | Gly | Gly |
| 150 | Ser | Ser | Ser | Ser |
| 151 | Ile | Ile | Leu | Leu |
| 152 | Ala | Ala | Ala | Ala |
| 153 | Ala | Ala | Leu | Pro |
| 154 | Arg | Arg | Arg | Arg |
| 155 | Val | Val | Val | Val |
| 156 | Ala | Ala | Ala | Ala |
| 157 | Thr | Thr | Arg | Arg |
| 158 | Gln | Gln | Gln | Gln |
| 159 | Leu | Leu | Leu | Leu |

The amino acid sequences shown in Table 2 were
each coupled to keyhole limpet haemocyanin and their
antigenicities determined by preparing antisera in
guinea pigs, which were then assayed for their ability
to neutralize FMDV by mixing virus with an equal volume
of normal or anti-serum and injecting 0.03 ml of the mixture into groups of 7 day old mice, as described in FEBS Letters, Vol. 157 No. 2, p 261-264 (1983).

The results are shown in Table 3, which shows the effectiveness of antibodies elicited by each of the amino acid sequences shown in Table 2 against viruses of types A10, A12A and A12B.

TABLE 3

| Amino Acid Sequence | Virus type | | |
|---|---|---|---|
| | A10 | A12A | A12B |
| A10 | +++ | − | − |
| A12A | − | +++ | + |
| A12B | − | + | +++ |
| Epicylin AP/A10/A12 | +++ | ++ | ++ |

+++ = excellent neutralization

++ = good neutralization

+ = moderate neutralization

− = no neutralization

In Epicylins $P/O_1K/C3$ and AP/A10/A12, it is, of course, possible to replace one or more of the amino acid residues thereof by other acid residues; especially where the residues are in the protein core and thus can be expected to have no effect on antigenicity. Thus, antigenic equivalents of these sequences also form part of the present invention.

Examples of other sequences in accordance with the invention and derived from FMDV VP1 protein are given below. In these sequences:

X represents a deletion;

parentheses indicate that the amino acid(s) immediately preceding them may optionally be replaced by the amino acid(s) within them;

"+" indicates that the two or more consecutive acids joined by "+" signs may together be replaced by the acid(s) in parentheses immediately following them;

/..../ indicates the position in a native protein of the amino acid to its immediate left.

## EPICYLIN P/A12/C3

Arg-Gly-Asp-Phe-Gly-Ser-Leu-Pro-Arg/C3-143; A12-154/-Val(Ala)-Ala-Arg/C3-145;A12-157/-Ala(X)-Gly/C3-146/-Asp-Leu-Ala-His-Leu-Ala-Ala-Ala-His-Ala-Arg-His-Leu/C3-159/-Pro

EPICYLIN AP/C3/A12

Arg-Arg/C3-145/-Gly-Asp-Leu-Ala-His-Leu-Ala-Ala-Ala-His-
Ala-Arg-His-Leu/C3-159/-Ala(Asp or Glu)-Asp(Glu or Ala)-
Phe/A12-148/-Gly-Ser-Leu-Ala-Pro-Arg-Val-Ala-Arg-Gln(X)-
Leu(X)/A12-159/


EPICYLIN AP/A10/C3 - 1

Leu(Ala)/A10-159/-Gln-Thr-Ala-Arg(Val)-Arg/A10-154;
C3-145/-Ala(Gly)/A10-146/-Asp-Leu(Ile or Ala)-
Ser/A10-150/-His/C3-150/-Leu-Glu+Ala (or Ala+Glu)-
Ala-His-Ala-Arg-His-Leu(Ala)/C3-159/-Arg(Ala)-Ala(X)
Alternatively for first six positions:

Leu-Gln-Thr-Arg-Val(Ala)-Arg/A10-154/-


EPICYLIN AP/A10(A12)/C3 - 2

This synthetic sequence is terminated at both ends by
the carboxyl ends of the A10 and C3 sequences. It is
possible to perform two separate syntheses and link the
C3 Arg-143 to A10 Gly-146. Alternatively part of A10
146 may be synthesised with opposite polarity from
Gly-146 to Leu-159. This polypeptide should also have
A12 activity.

Leu(Ala)/A10-159/-Gln-Thr-Ala-Val-Arg-Ala-Ala-Ile-Ser-Gly-Leu-Asp-Gly-Arg/A12-145; C3-143/-Ser(X)-Arg/C3-145/-Gly-Asp/C3-147/-Leu-Ala-His-Leu-Ala-Ala-Ala-His-Ala-Arg-His-Leu/C3-159/

## EPICYLIN P/A10(A12)/C3

Arg/A10-143/+Ser(X+Arg/C3-143/)-Arg/A12-145/-Gly/A10-146/-Asp/C3-147/-Glu(Asp)+Ala-[Leu+Glu(Asp)]-His-Leu/C3-151/-Ala-Ser/A10-150/-Ala(Ile)-His-Ala-Arg/C3-157; A10-154/-His-Leu(Ala)-Thr-Gln-Ala

## EPICYLIN P/SAT 1/SAT 2 - 1

Arg/SAT2-145/-Pro-Arg/SAT1-145; SAT2-insert/- Glu/SAT2-insert/-Asp/SAT1-147/-Val(Leu or Ala)/SAT1-148/-Ala-Thr/SAT1-150/(Gln/SAT2-150/)-Leu-Ala-Ala-Arg-Ile(Tyr)-Ala(Ser)-Glu(Ser or Ala)-Thr(Lys)-His-Ala(Leu or Ile or Ser)

## EPICYLIN P/SAT1/SAT2 - 2

Pro/SAT1-144/-Arg-Gly-Asp-Leu-Ala-Thr-Leu-Ala-Ala-Arg/SAT1-154; SAT2-insert/-Arg/SAT2-145/-(Ile/SAT1-146/-Ala-Ser-Glu/SAT1-insert; SAT2-insert/-Thr(Val)-His(Leu)-Ile/SAT1-159/(Ala)-Gln/SAT2-150/-Lys-Tyr/SAT2-152/-Ser-Ser-Ala-Lys-His-Ser-Leu(Ala)/SAT2-159/

EPICYLIN P/SAT1/$O_1$K

Pro/SAT1-144/(Leu/$O_1$-144/)-Arg-Gly/$O_1$-146/-Asp-
Leu(Ala)-Gln-Thr(Val)-Leu-Ala-Gln-Arg(Lys)-Ile-Ala-
Arg-Thr+Glu(Glu+Thr)-His/SAT1-158/-Leu

EPICYLIN AP/SAT1/$O_1$K

Ala-Arg/$O_1$-145/-Glu(Thr/SAT1-157/)-Asp(Glu)-Ser-Gln-
Val/$O_1$K-150(Ile/SAT1-155/)-Arg-Ala-Gln-Lys /$O_1$K-
154/(Leu or Ala)/-Thr-Ala-Arg/$O_1$K-157/(Lys)-Asp/SAT1-
147/(Glu or Thr)-Gly-Arg-Pro

EPICYLIN AP/$O_1$K/A10(A12)

Leu-Arg-Gly/$O_1$K-146/-Asp-Leu-Gln-Val-Leu-Ala-Gln-Lys-
Val-Ala-Arg/$O_1$K-157; A10-154/-Thr-Ala/A10-153/-Ala(X)-
Ile(Ala)-Ser-Gly-Leu-Asp-Gly-Ser+Arg(Arg + Ser + Arg)

EPICYLIN P/A10(A12)/$O_1$K

Arg/A10-143; $O_1$K-145/+Ser+Arg(Arg + Ser + X)-Gly-
Asp/A10-147; $O_1$K-147/-Leu(Ala or Ile)-Gln-Thr-
Val(Ser)-Leu(Ile)-Ala-Gln-Arg(Lys)-Val(Ala)-Ala-Arg-Gln-
Ala(Leu)

Other sites from the 130-144 region may be built in e.g.:

<u>EPICYLIN P/A12/0$_1$C (BFS)</u>

Arg-Gly-Asp-Phe-Gly-Ser-Leu-Ala-Pro-Arg/0$_1$C-135/-Val-
Ala-Arg/A12-157/-Gly(X)-Asn(Gln)/0$_1$C-139/-Ala-Ser-Gly

<u>EPICYLIN P/SAT2/A10</u>

Ile-Arg-Gly-Asp/SAT2-147/-Arg-Glu-Val-Leu/SAT2-148/-Ala-
Asn/A10-134/(Gln/SAT2-150/)-Lys/SAT2-151/-Tyr-Ser-
Ala(Ser)-Ser(Ala)-Asp(Lys)-Ser(His)-Arg-Ser-Pro

Sequences are also constructed to include another
antigenic region (200-213) of the viral capsid protein
e.g.:

<u>EPICYLIN P/C3/0$_1$</u>

C3 residues 144-153 and then Ala-His-Ala-Arg/C3-157:
0$_1$-201/-His/C3-158; 01-202/-Lys-Gln-Lys-Ile-Val-
Ala-Pro/0$_1$-209/

<u>EPICYLIN P/A10/0$_1$K</u>

Arg+Ser+Arg(Arg+Ser+X)-Gly-Asp-Leu-Gly-Ser-Ile-Ala-
Ala-Arg/A10-154; 0$_1$K-201/-His(Tyr or Val)-Lys-Thr-
Gln/A10-158; 0$_1$K-204/-Lys-Ile-Val-Ala-Pro

EPICYLIN P/SAT2/O$_1$K

Arg/SAT2-145/-Gly-Asp-Arg-Glu-Val-Leu-Ala-Gln-Lys-Tyr-Ser-
Ser-Ala-Lys/SAT2-156/-His-Ser/SAT2-158/(Gln)-Lys-Ile-Val-
Ala-Pro/O1-209/

Although this invention is described principally with reference to FMDV it may be of use for other viruses, e.g. polio virus, whose antigenic sites may be represented as overlapping sites on the surface of a helix. The advantage of the method is that a single polypeptide only need be synthesised instead of the two or more normally required to produce the antibody response to two or more antigenically distinct strains. The invention has been shown to be of value with the antigenic regions of the capsid proteins of picornaviruses (of which FMDV and polio virus are examples). It may also be applied to such other viruses as those of influenza, hepatitis, viral hog cholera, rabies, measles, rubella, mumps or (should it ever again become a problem) smallpox.

Polypeptides or other compounds comprising the amino acid sequences of the invention may be formulated in a conventional manner with carriers or adjuvants and are preferably formulated for injection, although other routes of administration are possible.

A further advantage of the invention in common with all synthetic polypeptide vaccines is that contamination with potentially infective material is avoided.

Another advantage is that, it is relatively easy to clone a synthetic DNA sequence corresponding to the amino acid sequence of the invention into a vector such as pBR322, since this plasmid readily accepts short lengths of DNA, and growth in E.coli HB101 or similar would allow expression of the polypeptide, which could be further enhanced by inclusion of promoters in the DNA sequence and other published procedures.

Since the polypeptide is small, and helical structures may readily be disrupted and reformed in vitro, spontaneous formation of the helical secondary structure is assured.

The polypeptide can be prepared according to any conventional method of synthesising peptides. Either solid phase processes or liquid phase processes may be employed, with the latter being advantageous in many cases. Such processes for synthesising peptides are described in, for example, "The Peptides", vol. 1 (1966) written by Schroder and Luhke (Academic Press, New York, U.S.A.) or "Peptide Synthesis" written by Izumiya et al., Maruzen Co. Ltd. (1975), and there are illustrated, for example, an azide process, a chloride process, an acid anhydride process, a mixed acid anhydride process, a DCC process, an active ester process, a process using Woodward reagent K, a carbodiimidazole process, an oxidation-reduction process, a DCC/additive (e.g. HONB, HOBt, HOSu, etc.) process, and the like.

In particular, the polypeptide or its amino acid sequence can easily be synthesised by conventional processes for synthesising polypeptides, especially the so-called stepwise process in which amino acids are condensed one by one onto a terminal amino acid, or by a process of coupling several fragments. More specifically, the above-described peptide can be prepared by condensing a reactive carboxyl group-containing starting material corresponding to one of the two fragments formed by separating the peptide at an arbitrary bond position with another reactive amino group-containing starting material corresponding to the other fragment in a manner conventionally employed for the synthesis of peptides and, where the resulting condensate contains a protective group or groups, removing the protective group(s) in a conventional manner.

The synthetic peptides of this invention may be used for treatment, prevention or diagnosis of viral infections of mammals including man. Vaccines of the peptides may be formulated and used in a conventional manner (see for example European Patent Publication No. 0 090 581 and U.K. Patent Specification No. 1 083 815).

Advantageously, the peptides are administered sub-cutaneously or intramuscularly at doses of between 1 to 100 microgram per kilogram body weight of the mammal, preferably from 10 to 50 microgram/kilogram. Maximum protection is likely to be afforded by a plurality of administered doses spaced from 2 to 8 weeks apart.

The invention is illustrated by the following Example, which should not be construed as a limitation thereof, and which illustrates the preparation of Epicylin $P/O_1K/C3$.

EXAMPLE

1.  Construction of Plasmid pXY413

The methods used for plasmid construction and analysis are described by Maniatis et al. [Molecular Cloning, A laboratory manual, Cold Spring Harbor Laboratory, Cold Spring Harbor, New York (1982)]. Techniques useful for the analysis of ß-galactosidase are described by J. Miller [Experiments in molecular genetics, Cold Spring Harbor Laboratory, Cold Spring Harbor, New York (1972)].

0114759

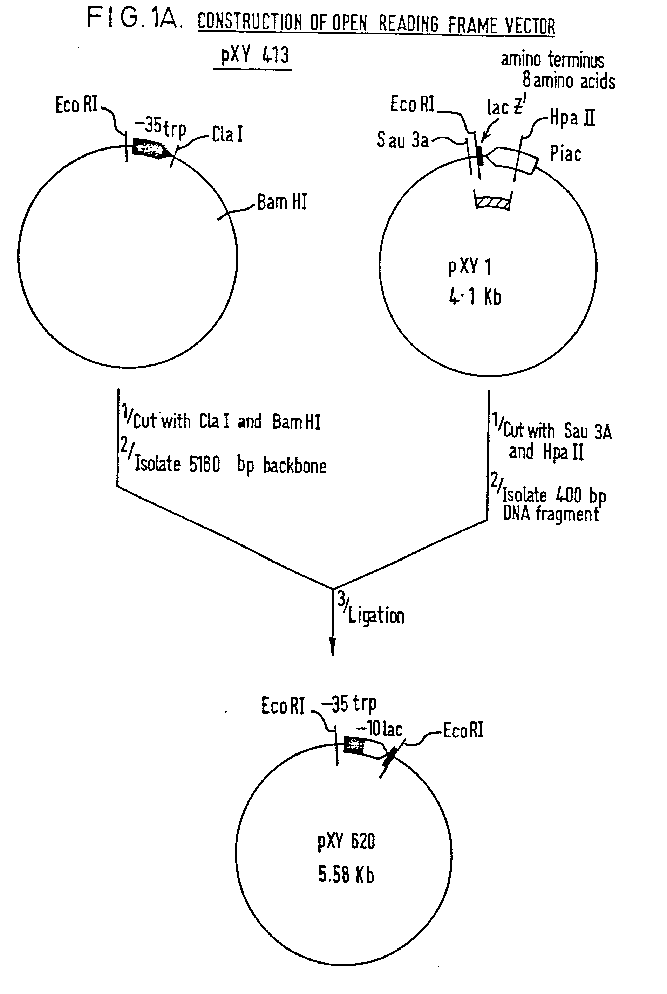

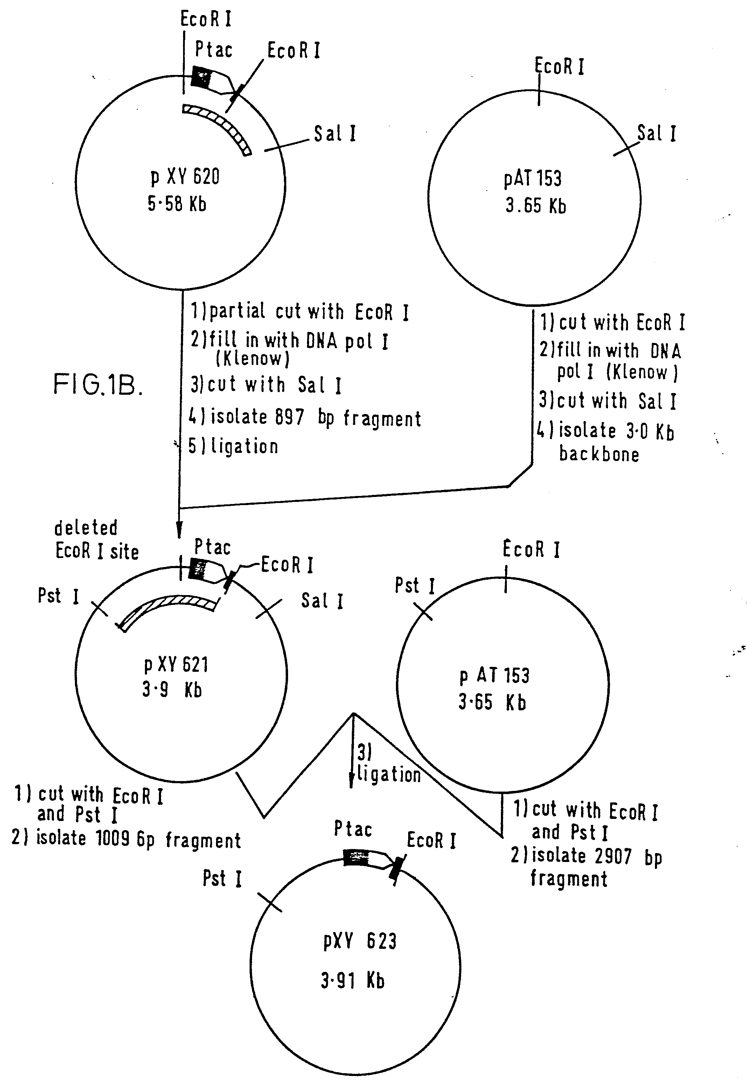

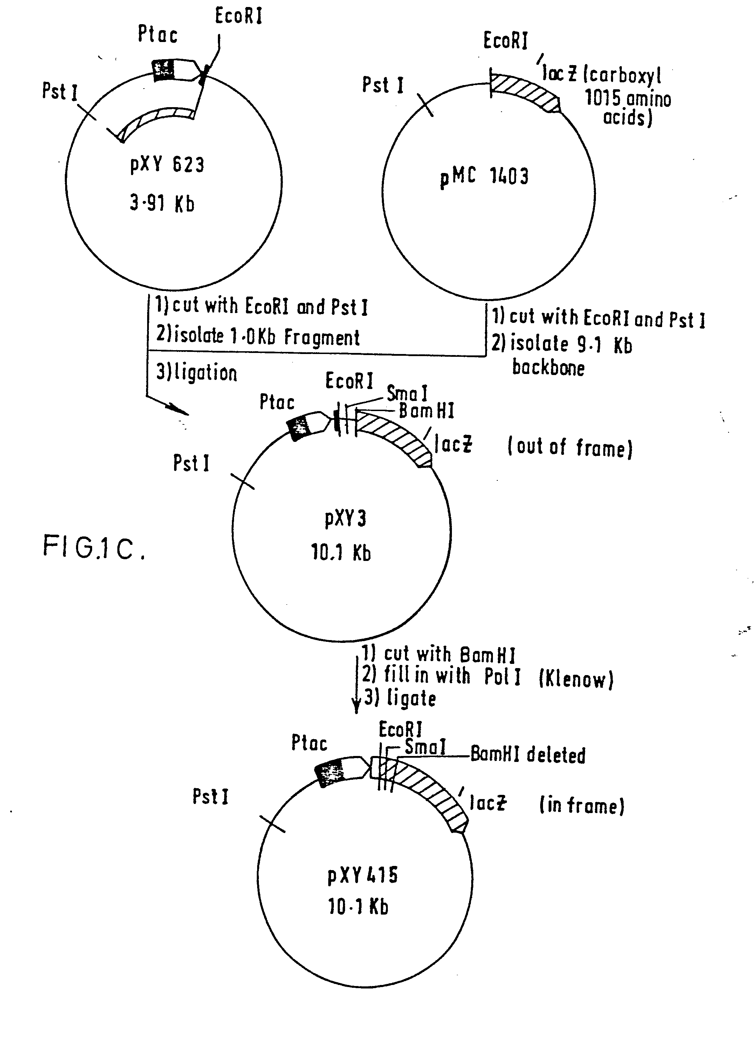

Plasmid pXY413, an open reading frame vector, was constructed as shown in Figure 1 of the accompanying drawings. The three starting plasmids were pEA300 obtained from Dr. Egon Aman (Harvard University, Cambridge MA, U.S.A.), pMC1403 [M. Casadaban et al., J. Bact. 143, 971 (1980)], and pOP203-13 obtained from Dr. F. Fuller (Harvard University, Cambridge MA, U.S.A.)

The lac genetic elements of plasmid pOP203-13 were recloned from the original pBR322 replicon onto a pAT153 replicon and renamed pXY1. This plasmid contains the lac promoter and operator elements and the first eight amino acids of ß-galactosidase. The -35 region of the trp promoter was provided by pEA300 and the hybrid tac promoter (pXY620) was constructed as shown in Figure 1A. To enhance its usefulness in subsequent plasmid constructions, the tac promoter and associated eight amino acids of ß-galactosidase were recloned onto pXY621 and subsequently pXY623 as shown in Figure 1B.

The plasmid pXY623 was then cut with PstI and EcoRI and separated by agarose gel electrophoresis. A 1000 base pair piece of DNA was isolated which contained the tac promoter ribosome binding site and start codon. This was ligated into plasmid pMC1403 which had also been cut with PstI and EcoRI. The resulting plasmid pXY413 contained the tac promoter and ribosome binding site, eight amino acids of ß-galactosidase, a polylinker

region, the enzymatically active carboxy terminal 1015 amino acids of ß-galactosidase, and a ß-lactamase gene and origin of replication for plasmid selection and maintenance (Fig. 1C).

Plasmid pXY413 does not direct the production of large quantities of ß-galactosidase under tac promoter control as the ribosomes which initiate translation at the ATG codon immediately downstream of the ribosome binding site are not in the same reading frame as the structural gene for ß-galactosidase. When plasmid pXY413 is transformed into a lac Z host (such as JM103 or JM105) the colonies are white on X-gal indicator plates indicating little or no ß-galactosidase activity is present.

As a test system for expression, the BamHI site of pXY413 was filled in with DNA polI (Klenow fragment) to adjust the reading frame and allow ß-galactosidase production from the tac promoter. An example of such a construction, pXY415, when transformed into JM105 produces large amounts of ß-galactosidase and gives blue colonies on X-gal plates. This is an important feature of pXY413 in that desired recombinants may be easily selected for by identifying blue colonies on X-gal indicator plates.

## 2.   Construction of Plasmid pWRL240

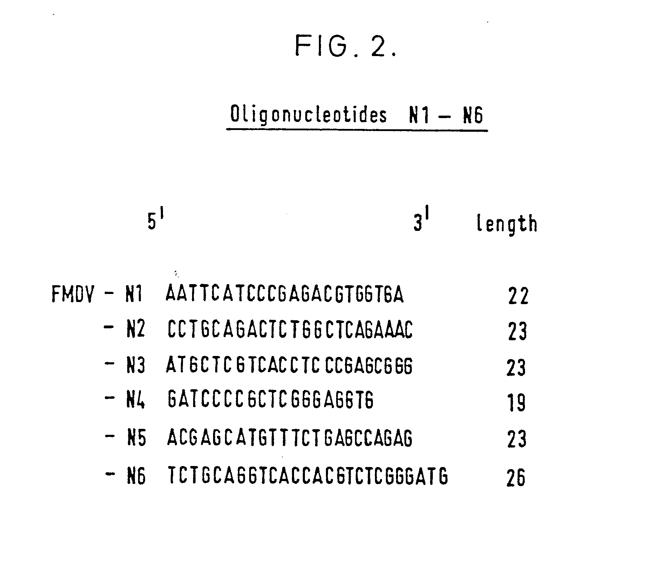

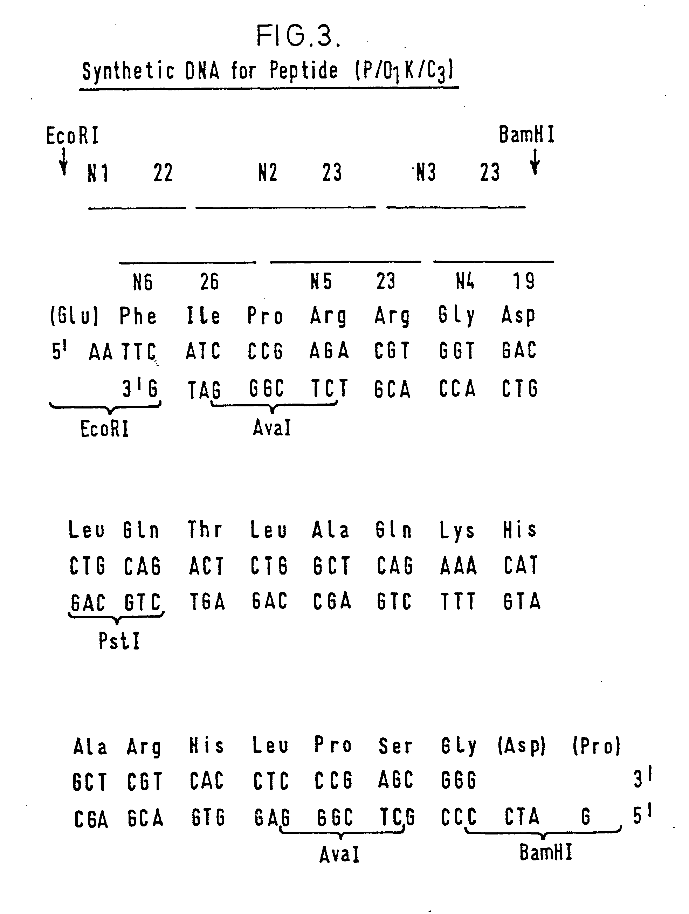

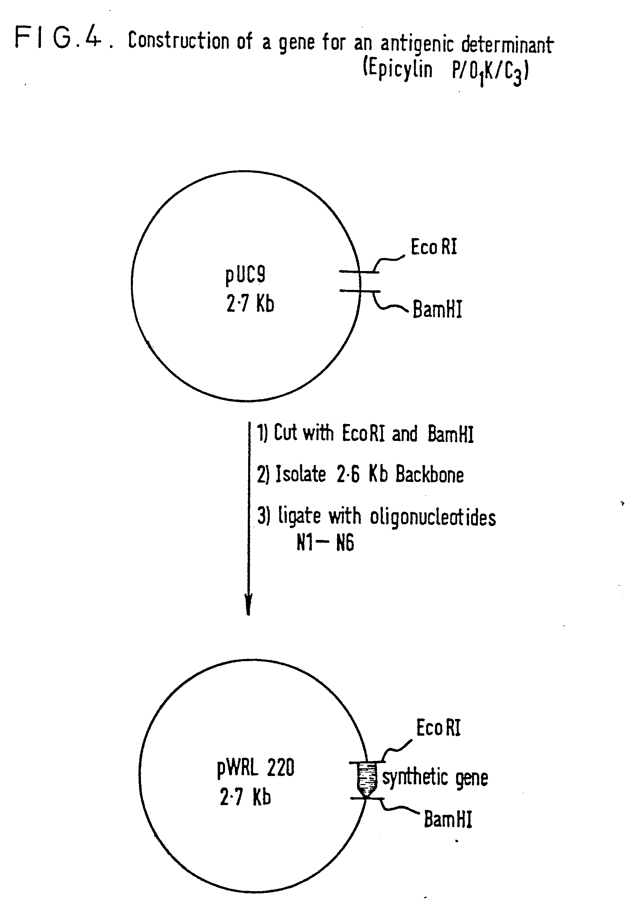

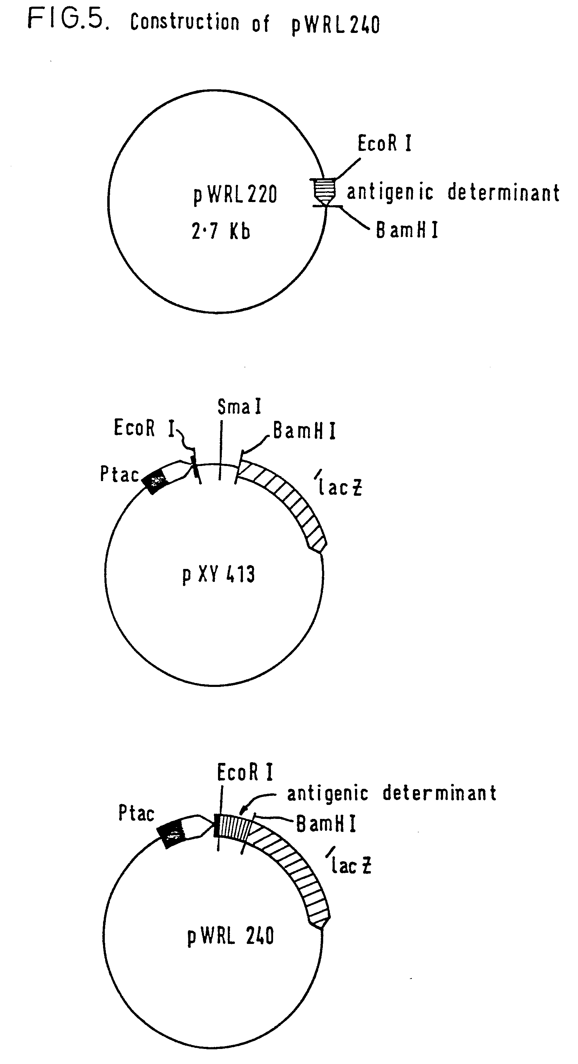

A synthetic gene coding for a hybrid FMDV VP1 antigenic determinant (serotypes $O_1$ and C3) - this is the material which I have named Epicylin P/$O_1$K/C3 - was assembled from six oligonucleotides, N1-N6 (Figure 2), cloned into pUC9 (Figure 4). After confirming that the resulting plasmid pWRL220 contained the desired sequence (Figure 3), the synthetic gene was removed by cutting the plasmid with EcoRI and BamHI and ligating the resulting 68 base pair fragment into the open reading frame vector pXY413 to construct plasmid pWRL240 as shown in Figure 5.

## 3.   Expression and antigenic activity of hybrid protein

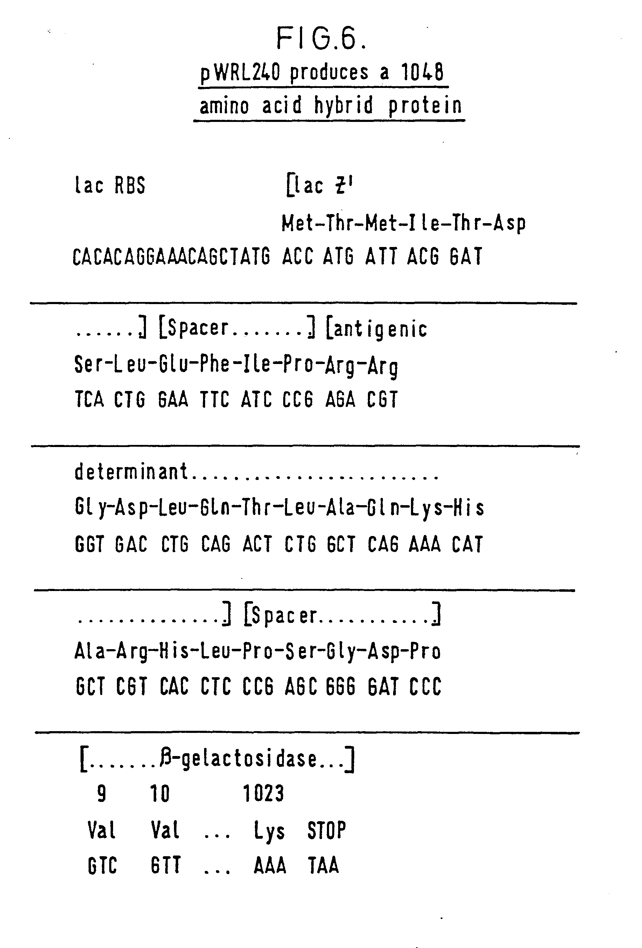

When transformed into JM105, pWRL240 directed the synthesis of high levels of a hybrid ß-galactosidase gene and gave blue colonies on X-gal indicator plates. The hybrid protein has 19 amino acids of VP1 fused to the amino-terminus of ß-galactosidase (Figure 6).

The hybrid protein produced by pWRL240 had the same specific activity as normal ß-galactosidase and can be purified by standard techniques (Miller, op. cit.) or affinity chromatography with antibodies to ß-galactosidase. Small amounts of the hybrid protein were purified by affinity chromatography and injected into Guinea Pigs together with adjuvant for testing of

immunogenic activity.

CLAIMS:

1.   A molecule comprising an amino acid sequence having at least two antigenic regions, each said region being capable of being recognized by antibodies raised against a viral protein sequence, each said region being formed of a chain of amino acids of said sequence, the chains forming said regions having at least one amino acid of said sequence in common, so that the chains at least partly overlap and are not contiguous along the length of the molecule.

2.   A molecule according to Claim 1, in which the sequence is such that it adopts a helical conformation.

3.   A molecule according to Claim 1 or Claim 2, which is a polypeptide.

4.   A molecule according to any one of the preceding Claims, in which said sequence is coupled to a larger compound to form a single molecule.

5.   A molecule according to Claim 4, in which said larger compound is keyhole limpet haemocyamin, bovine serum albumin, btg, ß-galactosidase or penicillinase.

0114759

6. A molecule according to any one of the preceding Claims, in which said overlap is such that at least 25% of the amino acids in one of said chains form part of the other said chain.

7. A molecule according to Claim 6, in which at least 50% of the amino acids in said one chain form part of said other chain.

8. A molecule according to Claim 7, in which at least 75% of the amino acids in said one chain form part of said other chain.

9. A molecule according to any one of the preceding Claims, in which said sequence is such that it adopts a helical conformation.

10. A molecule according to Claim 1, in which said sequence comprises the amino acids: Arg-Arg-Gly-Asp-Leu-Gln-Thr-Leu-Ala-Gln-Lys-His-Ala-Arg-His-Leu or an antigenic equivalent thereof.

11. A molecule according to Claim 1, in which said sequence comprises the amino acids: Arg-Ser-Arg-Gly-Asp-Leu-Gly-Ser-Ile-Ala-Ala-Arg-Val-Ala-Thr-Gln-Leu or an antigenic equivalent thereof.

12. A molecule according to any one of the preceding Claims, having the antigenicity of two or more different strains or subtypes of foot and mouth disease virus.

13. A molecule according to Claim 12, in which said strains are $O_1K$ and C3.

14. A molecule according to Claim 12, in which said strains are A10 and A12.

15. A vaccine comprising a molecule according to any one of the preceding Claims together with a carrier or adjuvant.

16. A synthetic peptide comprising a sequence of amino acids providing at least two antigenic sites, the amino acid sequence giving rise to a first antigenic site being interpolated with other amino acids forming at least part of a second antigenic site so that the amino acids of each site are conformationally contiguous and the peptide is capable of being recognised by two antisera separately raised against different viral strains.

17. A synthetic peptide as claimed in Claim 16, wherein the interpolation of amino acid comprises the sharing of one amino acid by the two antigenic sites.

18. A vaccine comprising a peptide as defined in Claim 16 or 17 together with an adjuvant or carrier therefor.

19. A vaccine as claimed in Claim 18, wherein the carrier is covalently linked to the peptide.

20. A vaccine according to any one of Claims 15 to 19, formulated for injection.

FIG.1A. CONSTRUCTION OF OPEN READING FRAME VECTOR pXY 413

EcoRI  —35trp  ClaI

BamHI

amino terminus
8 amino acids

EcoRI  lac Z'  HpaII
Sau 3a  Piac

pXY 1
4·1 Kb

1/ Cut with ClaI and BamHI
2/ Isolate 5180 bp backbone

1/ Cut with Sau 3A and HpaII
2/ Isolate 400 bp DNA fragment

3/ Ligation

EcoRI  —35 trp
—10 lac  —EcoRI

pXY 620
5.58 Kb

FIG.1B.

2/8

0114759

FIG.1C.

pXY 623
3·91 Kb

pMC 1403

1) cut with EcoRI and Pst I
2) isolate 1·0 Kb Fragment
3) ligation

1) cut with EcoRI and Pst I
2) isolate 9·1 Kb backbone

Ptac   EcoRI  Sma I
              BamHI
       lacZ   (out of frame)
Pst I

pXY3
10.1 Kb

1) cut with BamHI
2) fill in with Pol I (Klenow)
3) ligate

Ptac   EcoRI
       Sma I   BamHI deleted
       lacZ    (in frame)
Pst I

pXY 415
10·1 Kb

# FIG. 2.

### Oligonucleotides  N1 – N6

|  | 5' | 3' | length |
|---|---|---|---|
| FMDV – N1 | AATTCATCCCGAGACGTGGTGA | | 22 |
| – N2 | CCTGCAGACTCTGGCTCAGAAAC | | 23 |
| – N3 | ATGCTCGTCACCTC CCGAGCGGG | | 23 |
| – N4 | GATCCCCGCTCGGGAGGTG | | 19 |
| – N5 | ACGAGCATGTTTCTGAGCCAGAG | | 23 |
| – N6 | TCTGCAGGTCACCACGTCTCGGGATG | | 26 |

# FIG.3.

### Synthetic DNA for Peptide (P/O$_1$K/C$_3$)

```
EcoRI                                        BamHI
 ↓   N1      22          N2      23      N3     23  ↓
     _____     _____     _____


        N6      26         N5      23      N4     19
  (Glu)  Phe  Ile  Pro  Arg  Arg  Gly  Asp
  5'  AA TTC  ATC  CCG  AGA  CGT  GGT  GAC
          3'G  TAG  GGC  TCT  GCA  CCA  CTG
  _____/  _____/
      EcoRI               AvaI


  Leu  Gln  Thr  Leu  Ala  Gln  Lys  His
  CTG  CAG  ACT  CTG  GCT  CAG  AAA  CAT
  GAC  GTC  TGA  GAC  CGA  GTC  TTT  GTA
  _____/
      PstI


  Ala  Arg  His  Leu  Pro  Ser  Gly  (Asp)  (Pro)
  GCT  CGT  CAC  CTC  CCG  AGC  GGG              3'
  CGA  GCA  GTG  GAG  GGC  TCG  CCC  CTA   G    5'
                      _____/  _____/
                           AvaI              BamHI
```

FIG.4. Construction of a gene for an antigenic determinant
(Epicylin P/$O_1$K/$C_3$)

pUC9
2·7 Kb

EcoRI

BamHI

1) Cut with EcoRI and BamHI

2) Isolate 2·6 Kb Backbone

3) ligate with oligonucleotides N1— N6

pWRL 220
2·7 Kb

EcoRI

synthetic gene

BamHI

FIG.5. Construction of pWRL240

pWRL220
2·7 Kb

EcoR I

antigenic determinant

BamHI

Sma I

EcoR I

Ptac

BamHI

lacZ

pXY413

EcoR I

antigenic determinant

Ptac

BamHI

lacZ

pWRL 240

# FIG.6.
## pWRL240 produces a 1048
## amino acid hybrid protein

lac RBS            [lac Z'

                Met-Thr-Met-Ile-Thr-Asp

CACACAGGAAACAGCTATG ACC ATG ATT ACG GAT

---

    ......] [Spacer.......] [antigenic

Ser-Leu-Glu-Phe-Ile-Pro-Arg-Arg

TCA CTG GAA TTC ATC CCG AGA CGT

---

determinant........................

Gly-Asp-Leu-Gln-Thr-Leu-Ala-Gln-Lys-His

GGT GAC CTG CAG ACT CTG GCT CAG AAA CAT

---

    ...............] [Spacer...........]

Ala-Arg-His-Leu-Pro-Ser-Gly-Asp-Pro

GCT CGT CAC CTC CCG AGC GGG GAT CCC

---

    [.......β-gelactosidase...]

   9     10      1023

  Val   Val  ...  Lys  STOP

  GTC   GTT  ...  AAA  TAA